# EUROPEAN PATENT APPLICATION

(11) **EP 4 016 524 A1**
(43) Date of publication of application: **22.06.2022**
(21) Application number: 19941680.1
(22) Date of filing: 15.08.2019
(51) Int. Cl.: G10L 15/00

(54) **MEDICAL DEVICE AND MEDICAL DEVICE SYSTEM**

(71) Applicant: Shenzhen Mindray Bio-Medical Electronics Co., Ltd., Shenzhen, Guangdong 518057 (CN)
(72) Inventor: TAN, Lin, Shenzhen, Guangdong 518057 (CN)
(74) Representative: KIPA AB
(86) International application number: PCT/CN2019/100777
(87) International publication number: WO 2021/026881

(57) **Abstract**

A medical device (A, B, C). The medical device (A, B, C) is provided with an audio collector (101, 501, 602, 821) and a processor (103, 503, 604, 815). A user can operate, by means of a voice, the medical device (A, B, C) to execute a medical process comprising several medical actions (a, b, c). Specifically, the user inputs a voice signal carrying a medical process instruction to the medical device (A, B, C); after collecting the voice signal, the audio collector (101, 501, 602, 821) sends the voice signal to the processor (103, 503, 604, 815); the processor (103, 503, 604, 815) determines the corresponding medical actions (a, b, c) for the medical process instruction in the voice signal according to a preset correspondence between the medical process instruction and the medical actions (a, b, c), and controls execution of the medical actions (a, b, c). Hence, the user can operate the medical device (A, B, C) by using the voice, without manually performing a contact operation, so that the operating method is more efficient and convenient.

## Description

### TECHNICAL FIELD

The disclosure relates to the technical field of medical devices, and more particularly to a medical device and a medical device system.

### BACKGROUND

In clinical practice, medical staff can control medical devices to operate some medical procedures for patients, such as procedures related to monitoring and treatment. At present, the control process of medical device usually requires medical staff to approach the medical device and operate directly on the medical device manually.

However, this operation method has limitations in some specific medical scenarios. For example, in the aseptic operation scenario, manual contact with the medical device may lead to cross infection of bacteria. For another example, medical staff may not have time to manually operate the medical device in the process of emergency medical treatment. For another example, the medical device may be far away from the medical staff, so the medical staff cannot operate medical device at a close distance, and so on.

### SUMMARY

In this regard, a medical device for satisfying the demand of the medical staff to conveniently control the medical device, is provided in this disclosure.

In order to achieve the above purpose, following technical solutions are provided in the present disclosure.

In a first aspect, a medical device, is provided in this disclosure, which including:
an audio acquisition apparatus, which is operable to acquire a voice signal and to send the voice signal to a processor; wherein the signal carries a medical procedure instruction and is inputted by a user, the medical procedure instruction is operable to instruct the medical device to execute a preset medical procedure, wherein the medical procedure includes one or more preset medical actions, and there is a preset corresponding relationship between the medical procedure instruction and the medical action(s); and
the processor, which is operable to determine the medical action(s) that corresponds to the medical procedure instruction, and to control execution of the medical action(s), after receiving the voice signal.

In a second aspect, a medical device system, is provided in this disclosure, which including a first medical device and a second medical device, wherein
the first medical device is operable to acquire a voice signal which carries a medical procedure instruction, to determine a first medical action that corresponds to the medical procedure instruction and executed by the first medical device, and to execute the first medical action when an execution requirement of the first medical action satisfies a first preset requirement; and
the second medical device is operable to acquire the same voice signal which carries the medical procedure instruction, to determine a second medical action that corresponds to the medical procedure instruction and executed by the second medical device, and to execute the second medical action when an execution requirement of the second medical action satisfies a second preset requirement.

In a third aspect, a control device which is communicatively connected with at least one medical device, is provided in this disclosure, wherein the control device includes an audio acquisition apparatus, a communication interface and a processor;
wherein the audio acquisition apparatus is operable to acquire a voice signal, which carries a medical procedure instruction and is inputted by a user, and to send the voice signal to the processor;
the processor is operable to determine a medical action that corresponds to the medical procedure instruction, to determine a medical device that corresponds to the medical action, to obtain a communication address of the medical device, to generate a triggering command which triggers the medical device to execute the medical action, and to send the communication address of the medical device and the triggering command to the communication interface; and
the communication interface is operable to send the triggering command to the medical device according to the communication address of the medical device.

In a fourth aspect, a monitor is provided, which includes a sensor accessory, an audio acquisition apparatus, a processor and a display;
wherein the sensor accessory is operable to acquire physiological parameter data of a monitored object, and to send the physiological parameter data to the processor;
the audio acquisition apparatus is operable to acquire a voice signal which is inputted by a user and indicates to switch to a target monitoring mode, and to send the voice signal to the processor;
the processor is operable to process the physiological parameter data, to send the processed physiological parameter data to the display for displaying, to determine the target monitoring mode based on the voice signal, to determine configuration information that corresponds to the target monitoring mode, and to configure the monitor according to the configuration information; and
the display is operable to display the physiological parameter data which is processed by the processor.

### BRIEF DESCRIPTION OF THE DRAWINGS

In order to explain embodiments of this disclosure or technical solutions in the prior art more clearly, the following will briefly introduce drawings required in the description for the embodiments or the prior art description. It is obvious that the drawings in the following description are only some embodiments of this disclosure. For those skilled in the art, other drawings can be obtained from these accompanying drawings without paying any creative works.
FIG. 1 is a structural diagram of a medical device.
FIG. 2 is a diagram of controlling a medical device to execute a blood pressure zero calibration action by a voice signal.
FIG. 3 is a diagram of controlling a medical device system to operate by a voice signal.
FIG. 4 is another diagram of controlling a medical device system to operate by a voice signal.
FIG. 5 is another structural diagram of a medical device.
FIG. 6 is a structural diagram of a monitor.
FIG. 7 is an example diagram of pairing a mobile device with a monitor.
FIG. 8 is another structural diagram of a monitor.

### DESCRIPTION OF THE PREFERRED EMBODIMENTS

The technical solutions in example embodiments of the disclosure will be described clearly and completely below with reference to the accompanying drawings. Apparently, the embodiments described are merely some, rather than all, of the embodiments of the disclosure. It should be understood that the disclosure is not limited by the example embodiments described herein. All other embodiments derived by those skilled in the art without creative efforts on the basis of the embodiments described in the disclosure shall fall within the scope of protection of the disclosure.

Medical devices can execute related monitoring, treatment and other medical works under the control of medical staff. At present, the medical device is provided with entity keys which are corresponds to various functions. The medical staff controls the medical device by manually touching the entity keys to instruct the medical device to execute a medical procedure of certain functions.

However, this contact operation mode has great limitations in some medical scenarios, which are illustrated by several scenario examples below.

In aseptic operation scenarios, such as endotracheal intubation scenario, vascular puncture scenario and so on, the medical device used is not an aseptic device. The existing manual contact operation mode may cause the problem of cross infection of bacteria and have a negative impact on the patient's health. Therefore, at present, it often needs the cooperation of multiple medical staff, one of whom is responsible for operating the medical device, and the others are responsible for aseptic medical operation for patients. This treatment is a waste of human resources. Although some medical device is provided with foot treading devices to achieve the purpose of hand-operating aseptic operation device under the condition of reducing the number of cooperative personnel, however, this kind of medical device has many accessories, so is inconvenient for operation and cannot be popularly applied.

In the emergency medical procedure, such as cardiopulmonary resuscitation and out-hospital treatment and so on, the environmental conditions are usually complex, such as bumpy movement and narrow operation space. It may be difficult for medical staff to operate medical device manually. Moreover, in this scenario, the number of medical staff is often insufficient. The on-site medical staff pay full attention to the patient's condition and are busy treating the patient with both hands, so it may not be convenient to operate the device.

During the in-hospital transportation, such as hospital bed and wheelchair transportation or the out-hospital transportation, such as ambulance, helicopter, airliner, and lifeboat transportation and so on, the transportation process is relatively unstable, and the operation environment is complex. Contacting the keys on the operation device by the medical staff is very inconvenient and prone to misoperation.

The above medical scenarios are only used as examples. Of course, the medical device of this disclosure is not limited to the above three medical scenarios, but can also include other medical scenarios that restricts the manual control mode and can be thought of by those skilled in the art.

In order to solve the inconvenience caused by manual operation of medical device, this disclosure provides a medical device provided with an audio acquisition apparatus, and the user can control the medical device through voice instructions. It should be noted that in different application scenarios, the medical device can be specifically a monitor, a remote-control device, a ventilator, an infusion pump and other devices to realize various medical functions.

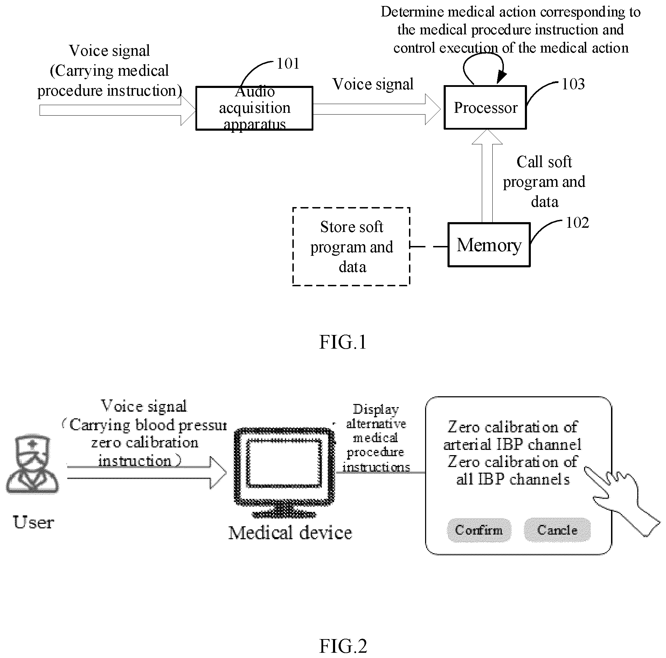

FIG.1 has shown a specific structure of a medical device. As show in FIG. 1, the medical device includes an audio acquisition apparatus 101, a memory 102 and a processor 103. It should be noted that the structure of the medical device is not limited to the above components. When the medical device is embodied as a device for realizing a specific function, it also includes a component unit for realizing the specific function.

The audio acquisition apparatus 101 is operable to acquire a voice signal which carries a medical procedure instruction and is inputted by a user and to send the voice signal to a processor; wherein the medical procedure instruction is operable to instruct the medical device to execute a preset medical procedure.

The medical device is provided with an audio acquisition apparatus which is operable to acquire a voice signal, specifically means that the audio acquisition apparatus of the medical device is operable to convert an analog voice signal into a digital signal that can be recognized by the medical device. In practical application, when a user needs a medical device to execute a medical function, he/she can input a medical procedure instruction to the medical device through a voice signal, and the audio acquisition apparatus acquires the voice signal which carries the medical procedure instruction and sends it to the processor. It should be noted that programs and data about one or more medical procedures are preset in the medical device. The corresponding medical procedure can be realized by calling the data and executing the program. The execution of the medical procedure can be considered to be equivalent to the realization of the corresponding medical functions. For example, when the medical device is specifically an infusion pump preset with a medical procedure of injecting drugs. The implementation of the medical procedure can be considered to realize the injection function of the infusion pump.

It can be understood that the user inputs the voice signal according to the actual medical needs, and the input time point of the voice signal is not fixed. In order to realize the real-time recording, the audio acquisition apparatus can be turned on based on an instruction to turn on the audio acquisition apparatus and kept in the working state to continuously detect whether the voice signal is received. Of course, based on the consideration of saving electric energy and computing resources of the medical device, the user can trigger to turn off the audio acquisition apparatus, or the medical device can automatically turn off the audio acquisition apparatus when the detection action of the audio acquisition apparatus satisfies a preset turning-off requirement. The preset turning-off requirement can include but are not limited to that no voice signal is detected for a certain period of time.

When the audio acquisition apparatus is turned off, if the user wants to carry out a medical operation procedure, he/she can turn on the audio acquisition apparatus through the instruction to turn on the audio acquisition apparatus to activate the voice control function of the medical device. Specifically, if the processor of the medical device receives the instruction to turn on the audio acquisition apparatus, the audio acquisition apparatus can be turned on. The ways in which the processor turns on the audio acquisition apparatus may include but are not limited to the following ways.

For example, the medical device can be provided with a physical key for activating the audio control function. The user can press the key, and then based on the press operation, the key generates the instruction to turn on the audio acquisition apparatus and sends it to the processor. The processor then turns on the audio acquisition apparatus to acquire the voice signal inputted by the user at any time.

As another example, the medical device can include a touch-sensitive display, which displays a software key for activating the audio control function. The user can click the software key to trigger the touch-sensitive display to generate the instruction to turn on the audio acquisition apparatus and sends it to the processor. After receiving the instruction to turn on the audio acquisition apparatus, the processor turns on the audio acquisition apparatus to acquire the voice signal inputted by the user at any time.

As another example, the medical device can include a touch-sensitive display. The user can input a preset gesture trace on the touch-sensitive display, such as drawing a circle or other graphics. After receiving the gesture trace, the touch-sensitive display sends it to the processor. If the processor determines that the gesture trace is a preset gesture trace for turning on the audio acquisition apparatus, it determines that the instruction to turn on the audio acquisition apparatus is received, and then turns on the audio acquisition apparatus to acquire the voice signal inputted by the user at any time.

For another example, the medical device can include a photoelectric sensor. The user inputs a light blocking action that satisfies a preset requirement through the photoelectric sensor. Specifically, for example, the user blocks the photoelectric sensor for a preset time, so that the photoelectric sensor can detect a change of light that satisfies the preset requirement. Then, the instruction to turn on the audio acquisition apparatus is generated and sent to the processor. The processor then turns on the audio acquisition apparatus to acquire the voice signal inputted by the user at any time.

For another example, the medical device can include an image acquisition apparatus. The user can give a gesture (such as drawing a triangle, a circle, etc.) that satisfies a preset requirement. The image acquisition apparatus can acquire an image containing the gesture and send the image to the processor. The processor can use an image processing technology to analyze content of the image. If the gesture that satisfies the preset requirement is detected from the image, it can be determined that the instruction to turn on the audio acquisition apparatus is received. The processor then turns on the audio acquisition apparatus to acquire the voice signal inputted by the user at any time.

As another example, the medical device may include a biological characteristic acquisition apparatus which is operable to acquire biological characteristic information of the user. In practical application, the biological characteristic acquisition apparatus can be any one of an iris acquisition apparatus, a fingerprint acquisition apparatus, a voiceprint acquisition apparatus and so on. The biological characteristic acquisition apparatus sends the acquired biological characteristic information to the processor, and the processor determines whether the user is corresponding to the biological characteristic information has the permission of turning on the audio acquisition apparatus. A specific determination method is as follows. The processor pre-stores the biological characteristic information of one or more users who have permission of turning on the audio acquisition apparatus and determines that whether the received biological characteristic information is the same as any of the pre-stored biological characteristic information. Only when the user who corresponds to the biological characteristic information has the permission of turning on the audio acquisition apparatus, the processor determines that it receives the instruction to turn on the audio acquisition apparatus, and then turns on the audio acquisition apparatus to acquire the voice signal inputted by the user at any time.

Furthermore, the medical device may include an image acquisition apparatus which is operable to acquire a barcode image inputted by the user, such as one-dimensional code, two-dimensional code, etc. The barcode image records identity information, such as name, department, position, etc., of the user. The image acquisition apparatus acquires the barcode image and sends it to the processor. The processor extracts the identity information from the barcode image and determines whether the user who corresponds to the identity information has the permission of turning on the audio acquisition apparatus. A specific determination method is as follows. The processor pre-stores the identity information of one or more users who have permission of turning on the audio acquisition apparatus and determines that whether the received identity information is the same as any of the pre-stored identity information. Only when the user who corresponds to the identity information has the permission of turning on the audio acquisition apparatus, the processor determines that it receives the instruction to turn on the audio acquisition apparatus, and then turns on the audio acquisition apparatus to acquire the voice signal inputted by the user at any time.

For another example, the medical device can be provided with a communication interface, which can receive the instruction to turn on the audio acquisition apparatus which is sent by other devices and send it to the processor. Other devices can be interconnected medical devices in the same medical system, or they can also be separate control node devices. The processor receives the instruction to turn on the audio acquisition apparatus and then turns on the audio acquisition apparatus to acquire the voice signal inputted by the user at any time.

For another example, the medical device can be provided with a turning-on key, and the user can trigger the key to turn on the medical device. If the medical device receives an instruction to turn on the medical device, the medical device turns on the audio acquisition apparatus during its powering up, to acquire the voice signal inputted by the user at any time.

For another example, when it is necessary to use the medical device to monitor the vital signs of a patient for the first time, the user can input an instruction to start monitoring to the medical device, as well as input related information of the monitored object to the medical device, such as name, age, gender, etc. After receiving the instruction to start monitoring, the processor can determine that the instruction to turn on the audio acquisition apparatus is received and then turn on the audio acquisition apparatus to acquire the voice signal inputted by the user at any time.

Of course, the above ways to turn on the audio acquisition apparatus are only examples, and other ways that can be thought of by those skilled in the art are within the protection scope of this disclosure. In addition, turning off the audio acquisition apparatus can also take any of the above methods, or can be implemented by inputting a voice signal with an instruction to turn off the audio acquisition apparatus.

The memory 102 is operable to store a software program and data.

Specifically, a software program for realizing the voice control function and its related data can be stored in the memory. The processor realizes the following voice control function by calling the software program and related data in the memory.

The processor 103 is operable to run the software program stored in the memory, call the data stored in the memory, and at least execute the following steps of determining the medical action that corresponds to the medical procedure instruction and controlling execution of the medical action, after receiving the voice signal.

After the audio acquisition apparatus sends the voice signal to the processor, the processor analyzes and processes the voice signal. Specifically, the processor extracts voice content from the voice signal in a digital form. The voice content is the medical procedure instruction inputted by the user. The medical procedure instruction is operable to instruct the medical device to execute a specific medical procedure. The medical procedure includes one or more preset medical actions which can be executed in serial, in parallel, or partially in serial and partially in parallel.

The corresponding relationship between the medical procedure instruction and the medical action can be preset in the medical device. After the processor extracts the medical procedure instruction from the voice signal, the medical action that corresponds to the medical procedure instruction can be determined according to the corresponding relationship. The medical action may be one or more. The processor controls the execution of the medical action. Specifically, the medical action may be executed by other components or accessories of the medical device, and the processor controls other components or accessories to accurately execute the medical action through an instruction. For example, the medical device is a monitor, and the medical actions determined by the processor includes displaying the monitoring interface which corresponds to a monitoring mode, then the processor sends the parameter data of the monitoring interface to the display to enable it to display the monitoring interface according to the parameter data. For another example, if the medical device is an infusion pump, and the medical action determined by the processor includes injecting a certain drug, then the processor controls the pump device of the infusion pump to inject the drug into the monitored object through an instruction. Of course, the medical action herein is only an example. In practical application, the medical action controlled and executed by the processor is not limited to this.

The following describes in detail how the processor determines the corresponding medical action according to the medical procedure instruction.

An instruction database can be preset in the medical device. The instruction database includes one or more medical procedure instructions. Different kinds of medical procedure instructions are operable to instruct the medical device to execute different medical procedures. It should be noted that the same kind of medical procedure instructions are only in different predefined formats, but they are all operable to instruct the medical device to execute the same medical procedure. For example, the instruction database includes three medical procedure instructions which are "endotracheal intubation", "endotracheal intubation for patient" and "entering an endotracheal intubation mode", and the three medical procedure instructions belong to the same kind of medical procedure instruction, and their purpose is to instruct the medical device to execute the medical procedure of endotracheal intubation.

An action database can also be preset in the medical device. The action database includes one or more medical actions. The medical procedure instruction in the instruction database has a preset corresponding relationship with the medical action in the action database. It can be understood that the medical action that corresponds to the same kind of medical procedure instruction are the same. One medical procedure instruction can be corresponding to one or more medical actions. The corresponding relationship can also indicate that the medical procedure indicated by the medical procedure instruction includes the one or more medical actions.

It should be noted that, one method for presetting the instruction database includes that the medical device is provided with an instruction database configuration function, so the user can input a user-defined name of the medical procedure instruction into the instruction database and can preset which medical action or medical actions the user-defined medical procedure instruction corresponds to. For example, according to a daily language habits, the user inputs a medical procedure instruction in the instruction database as "blood pressure zero calibration" and presets its corresponding medical action as "zero calibration of all IBP channels". Of course, according to daily usage habits, the medical procedure instruction also can be preset to correspond other medical action, such as "zero calibration of measurement channel of arterial blood pressure".

Another method for presetting the instruction database includes acquiring the name of the function key of the medical device as the name of the medical procedure instruction. In this way, the user only needs to input the voice signal according to the name of the function key, and the medical device can acquire the voice signal containing the medical procedure instruction. After presetting the medical procedure instruction according to the function key, the medical action executed by the medical device after triggering the function key is further determined, and then the medical action is preset as the medical action that corresponds to the medical procedure instruction. Of course, the above two methods can be used in combination.

In addition, the corresponding relationship between the medical procedure instruction and the medical action can be preset by self-learning. Specifically, in the self-learning mode, the processor acquires any voice signal with any medical procedure instruction through the audio acquisition apparatus, records the medical action executed by the medical device within a preset time before and after an input time point of the any voice signal, and then establishes the corresponding relationship between the any medical procedure instruction and the recorded medical action.

Specifically, the medical device can be preset to the self-learning mode. For example, the medical device can be preset to the self-learning mode at the beginning of its usage or can be manually operated to enter the self-learning mode at any other time. In the self-learning mode, the user can input the voice signal which carries the medical procedure instruction while operating the medical device. After acquiring the voice signal, the audio acquisition apparatus sends the voice signal to the processor. The processor records the medical procedure instruction in the voice signal, records the medical action executed by the medical device before and/or after receiving the medical procedure instruction, establishes the corresponding relationship between the medical procedure instruction and the medical action, and then obtains a learning result. The self-learning mode can last for a period of time, during which the processor can generate a group of corresponding relationship between the medical procedure instructions and medical actions after learning and training. In addition, the medical procedure instructions are stored in the instruction database and the medical actions are stored in the action database.

The medical procedure instruction has a predefined format. If the user inputs the voice signal according to the predefined format, the processor can accurately determine the same medical procedure instruction in the instruction database after extracting the medical procedure instruction from the voice signal, and then determine the medical action that corresponds to the medical procedure instruction from the action database.

However, different users have a variety of language habits. The medical procedure instruction carried by the voice signal may not fully comply with the predefined instruction format, or the medical procedure instruction in the instruction database are monotonous, and different expression formats are not preset for the same medical procedure instruction. In this way, in practical application, after the processor extracts the medical procedure instruction from the voice signal, it needs to find the medical procedure instruction which is similar to the extracted medical procedure instruction in the preset instruction database. The requirement for determining that the two medical procedure instructions are similar is that, after analyzing semantics of the two medical procedure instructions, the semantic similarity of the two medical procedure instructions satisfies a preset similarity requirement. The preset similarity requirement can be embodied as a preset similarity threshold. For facilitating description, the medical procedure instruction found from the preset instruction database can be called a target medical procedure instruction.

To sum up, the processor determines the medical action that corresponds to the medical procedure instruction, including the following steps: finding the target medical procedure instruction in the preset instruction database, wherein the target medical procedure instruction has a semantic similarity to the medical procedure instruction that satisfies the preset similarity requirement and determining a medical action in the preset action database which corresponds to the target medical procedure instruction. The first step is also called as an instruction matching process. It should be noted that the semantic similarity satisfies the preset similarity requirement, also includes the case that the two medical procedure instructions are exactly the same.

In order to improve the reliability of instruction matching results, the instruction matching process can interact with the user, and the user can select the desired target medical procedure instructions. Specifically, the processor searches for a medical procedure instruction has a semantic similarity to the medical procedure instruction that satisfies a first preset similarity threshold. Wherein the medical procedure instruction is extracted from the voice signal. If such medical procedure instruction is found, the found medical procedure instruction is determined as the target medical procedure instruction. Or else, the processor searches for a medical procedure instruction has a semantic similarity to the medical procedure instruction that satisfies a second preset similarity threshold, and outputs the found medical procedure instruction to the user and then receives a command for the user to select a medical procedure instruction, and finally determines the medical procedure instruction selected by the user as the target medical procedure instruction.

The first preset similarity threshold and the second preset similarity threshold can be preset according to the actual demand. The first preset similarity threshold is higher than the second preset similarity threshold. If a medical procedure instruction satisfying the first preset similarity threshold is found, it can be considered that the found medical procedure instruction is the medical procedure instruction expected by the user in the actual demand. If a medical procedure instruction that does not satisfy the first preset similarity threshold but satisfies the second preset similarity threshold is found, it can be considered that it may meet the user's expectations, and it will be displayed to the user through the interactive interface for selection. The search result displayed on the interactive interface may only include one medical procedure instruction, so the user can choose to confirm or cancel the operation. Or the search result includes multiple medical procedure instructions, and the user can select one medical procedure instruction as the target medical procedure instruction.

The above implementation will be described in detail in combination with FIG. 2. As shown in FIG.2, the user inputs a voice signal including "blood pressure zero calibration" to the medical device. After the medical device extracts the medical procedure instruction, it executes a semantic search in the preset instruction database, finds the medical procedure instructions that satisfy the second preset similarity threshold and include "zero calibration of arterial IBP channel" and "zero calibration of all IBP channels", and displays the search results on the interactive interface for the user to select. Assuming that the user selects "zero calibration of all IBP channels", the medical procedure instruction "zero calibration of all IBP channels" will be taken as the target medical procedure instruction.

Of course, other steps in the instruction matching process can also interact with the user. For example, after finding the medical procedure instruction that satisfies the first preset similarity threshold, it can be displayed to the user through the interactive interface to ask the user whether to execute the medical procedure instruction. The user can select a confirm option or a cancel option. After the option is cancelled, the user can adjust the format of the medical procedure instruction and input the voice signal again.

After matching the target medical procedure instruction in the preset instruction database, the corresponding medical action in the preset action database is searched for. The medical action may include a plurality of medical actions having a preset execution sequence. The processor first determines the execution sequence of each medical action, and then controls the execution of each medical action according to the execution sequence.

The plurality of medical actions may be executed serially. For example, the target medical procedure instruction matched by the processor of the monitor is "5-minute noninvasive blood pressure measurement". The medical actions which correspond to the target medical procedure instruction include "setting NIBP measurement interval as 5 minutes" and "starting NIBP measurement", and the execution sequence of the first medical action is preset as 1 and the execution sequence of the next medical action is preset as 2. The monitor first configures the interval of non-invasive blood pressure (NIBP) measurement as 5 minutes, and then automatically starts NIBP measurement every 5 minutes. For another example, the target medical procedure instruction matched by the processor of the monitor is "switching to large font interface". The medical actions which correspond to the target medical procedure instruction include "closing a current interface" and "displaying a large font interface", and the execution sequence of the first medical action is preset as 1 and the execution sequence of the last medical action is preset as 2. The monitor firstly closes the currently displayed interface, and then generates a large font interface and displays it. It should be noted that the execution sequence also includes a parallel execution.

The sequence of the medical actions can be preset in the system of the medical device or customized by the user through the configuration interface. After a plurality of medical actions are determined, the plurality of medical actions can be stored in a medical action queue to ensure that the plurality of medical actions are executed in sequence.

The execution process of the medical procedure can be fed back to the user, such as through a visual feedback to the user by the display interface or an auditory feedback to the user through an output sound, so as to make the user know that the medical action is being executed. Different medical actions output different feedback information. For example, during NIBP measurement, the monitor displays the measurement interval, measurement countdown and measured real-time pressure value on the display interface to inform the user that NIBP measurement is in progress. After the measurement, the monitor outputs an ending tone and displays the final measurement result. For another example, during endotracheal intubation, the monitor can suspend displaying all monitoring parameters of respiratory system, such as respiratory rate, end expiratory carbon dioxide, respiratory mechanics, etc., and display the indication information that "endotracheal intubation mode has been started". It should be noted that, the output information of the medical procedure can be configurated according to the actual needs and is not specifically limited in this disclosure.

It can be seen from the above technical scheme that this disclosure provides a medical device with an audio acquisition apparatus and a processor. The user can control the medical device through a voice signal to execute the medical procedure including several medical actions. Specifically, the user inputs the voice signal which carries the medical procedure instruction to the medical device, and the audio acquisition apparatus acquires the voice signal and sends it to the processor. The processor determines the corresponding medical action for the medical procedure instruction in the voice signal according to the preset correspondence between the medical procedure instruction and the medical action, and finally controls the execution of the medical action. It can be seen that users can use voice to control medical device without any manual contact operation, so as to avoid the problems caused by contact operation, and the control method is more efficient and convenient.

In practical application, the medical device can specifically be different forms of devices, such as a monitor, ventilator, anesthesia machine, infusion pump, and so on. On the basis of including the audio acquisition apparatus and processor, the medical device may also include other components. Different medical devices have different components. For example, the monitor can include an information output apparatus, such as a display. For example, the monitor can include physiological sign detection accessories, such as blood oxygen probe, and the infusion pump can include drug injection components, etc.

In different application scenarios, the specific contents of medical actions are also different. For example, a medical action may include any one or more of the following types: a medical action that triggers an information output apparatus to output related content of the medical procedure, a medical action that triggers a physiological sign detection accessory to execute a measurement, a medical action that triggers a drug injection component to inject a drug, and so on.

An example of an application scenario is that, supposing that the medical device is an infusion pump. Multiple infusion pumps operate at the same time, and the user controls the operation of a certain one infusion pump through a voice signal. Among them, the medical procedure instruction carried by the voice signal is an instruction for a target infusion pump to inject a target drug, wherein the target infusion pump refers to one specific infusion pump among the multiple infusion pumps. Then the audio acquisition apparatus of each infusion pump can acquire the voice signal and send it to their respective processors. Each processor implements following processing steps. The processor determines whether its own infusion pump is the target infusion pump after receiving the voice signal carries the instruction for a target infusion pump to inject a target drug. If yes, the processor determines a drug injection action that corresponds to the instruction for a target infusion pump to inject a target drug and controls execution of the drug injection action.

Among them, an instruction to inject a target drug, can include any one or more of the following: an instruction to stop injecting a target drug, an instruction to start injecting a target drug, an instruction to inject a target drug according to a target parameter. The target parameter can be specified as a target speed.

An example of another application scenario is that, supposing that the medical device is specifically an anesthesia machine, and the user instructs the anesthesia machine to operate through a voice signal. The anesthesia machine anesthetizes the patient by controlling related parameters of anesthesia gas and oxygen, such as concentration and flow rate. Among them, the medical procedure instruction carried by the voice signal is an instruction to inject a target anesthetic. The audio acquisition apparatus of the anesthesia machine acquires the voice signal and sends it to the processor. The processor implements following processing steps. The processor determines a drug injection action that corresponds to the instruction to inject a target anesthetic and controls execution of the drug injection action. Similarly, the instruction to inject a target anesthetic can include any one or more of the following: an instruction to stop injecting a target drug, an instruction to start injecting a target drug, an instruction to inject a target drug according to a target speed.

An example of another application scenario is that, supposing that the medical device is specifically a ventilator, and the user instructs the ventilator to configure a working mode or working parameter through a voice signal. The medical procedure instruction carried by the voice signal is an instruction to configure a target working mode, the audio acquisition apparatus of the ventilator acquires the voice signal and sends it to the processor. The processor implements following processing steps. The processor determines a mode configuration action that corresponds to the instruction to configure a target working mode and executes the mode configuration action. Or the medical procedure instruction carried by the voice signal is an instruction to configure a target working parameter, the audio acquisition apparatus of the ventilator acquires the voice signal and sends it to the processor. The processor implements following processing steps. The processor determines a parameter configuration action that corresponds to the instruction to configure a target working parameter and executes the parameter configuration action. Specifically, the working mode of the ventilator may include but is not limited to a sputum suction mode, a rescue mode or a ventilation mode. The ventilation modes can include a variety of modes, such as a continuous ventilation mode, an intermittent ventilation mode at a certain time interval, etc. The working parameters of the ventilator may include but are not limited to a pressure, a tidal volume, a respiratory rate, etc.

It should be noted that, the medical devices can be interconnected to form a medical device system. If the medical action determined by the processor of the medical device needs to be executed by other medical devices other than the medical device, the processor of the medical device determines the execution device which corresponds to each medical action. For example, the medical device which corresponds to the medical action can be recorded, and the processor determines the execution device which corresponds to the medical action according to the recorded information. Then, the processor of the medical device triggers each execution device to execute the corresponding medical action according to the execution sequence. The triggering method can be that when it is the time for executing a certain medical action according to the execution sequence, the processor of the medical device sends the content of the medical action to the execution device which corresponds to the medical action to trigger the execution device to execute the medical action.

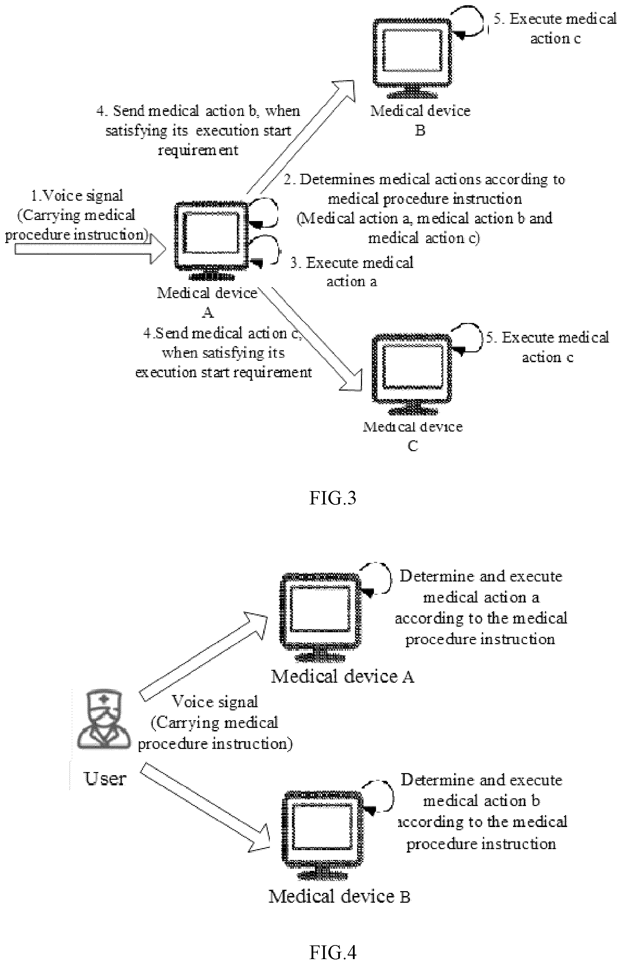

As shown in FIG.3, three medical devices are communicationally interconnected with each other. Medical device A is the medical device that receives a voice signal. Assuming that the processor of the medical device A determines three medical actions according to the medical procedure instruction in the voice signal. The three medical actions includes medical action a, medical action b and medical action c. The three medical actions need to be executed by medical device A, medical device B and medical device C respectively. Supposing that the execution sequence of the three medical actions is that the medical action a is prior than the medical action b and medical action c, and the medical action b is synchronized with medical action c. Then, the medical device A first executes the medical action a by itself, and then triggers the medical device B and medical device C to execute the medical action b and medical action c respectively when the execution start requirements of the medical action b and medical action c are satisfied. Of course, the execution sequence of the medical actions can be in other forms, the execution devices of the medical actions can also be in other forms. The execution start requirements of the medical action b and medical action c may be the same or different, and the time points at which the medical device B and medical device C execute their respective medical actions may be the same or different.

A specific application example is as follows. The monitor is communicationally connected with the anesthesia machine and infusion pump respectively. The monitor has an audio acquisition module. The user can input the medical procedure instruction to the monitor through a voice signal. For example, the medical procedure instruction carried by the voice signal is an instruction to inject one or more anesthetics after BIS (Bispectral index) exceeds a certain value. The monitor analyzes and processes the medical procedure instruction, determines that the medical actions which correspond to the medical procedure instruction include two medical actions of monitoring BIS value and injecting anesthetic, determines that the two medical actions need to be executed in sequence, and then determines that the first medical action is executed by the monitor itself, and the last medical action needs to be executed by the anesthesia machine and infusion pump. Therefore, the monitor firstly executes the medical action of monitoring BIS value. When the monitored BIS value reaches the value in the voice signal, it sends a triggering command to the anesthesia machine and infusion pump respectively to instruct the anesthesia machine and infusion pump to execute the medical action of injecting corresponding anesthetics respectively.

Another specific application example is as follows. The monitor is communicationally connected with a ventilator. The monitor has an audio acquisition module. The user can input the medical procedure instruction to the monitor through a voice signal. For example, the medical procedure instruction carried by the voice signal is an instruction to implement an endotracheal intubation. The monitor analyzes and processes the medical procedure instruction, determines that the medical actions which correspond to the medical procedure instruction include two medical actions of suspending breathing parameter monitoring and suspending ventilator ventilation, determines that the two medical actions can be executed at the same time, and determines that the first medical action is executed by the monitor itself and the last medical action is executed by the ventilator. Thus, the monitor executes the medical action of suspending breathing parameter monitoring and sends a triggering command to the ventilator to instruct the ventilator to execute the medical action of suspending ventilator ventilation. It should be noted that the medical actions which correspond to the medical procedure instruction of the endotracheal intubation also include switching to an endotracheal intubation mode after suspending breathing parameter monitoring, by the monitor. Therefore, after the monitor executes the medical action of suspending breathing parameter monitoring, it also configures its own mode to the endotracheal intubation mode.

In the above medical device system, the voice signal is acquired by a medical device in the system, and then analyzed and processed by the medical device. However, the medical devices in the system can all have their own audio acquisition apparatus and processor, and the medical devices can respectively realize the acquisition, analysis and processing of the voice signal.

Specifically, this disclosure further provides a medical device system, including a first medical device and a second medical device. Among them, "first" and "second" are only to distinguish different medical devices, and do not constitute restrictions on the execution sequence of medical actions and other contents. Moreover, the numbers of the first medical device and the second medical device are not limited to one, but can also be multiple.

The first medical device is operable to acquire a voice signal which carries a medical procedure instruction, to determine a first medical action that corresponds to the medical procedure instruction and executed by the first medical device, and to execute the first medical action when execution requirement of the first medical action satisfies a preset requirement.

The second medical device, which is operable to acquire the voice signal which carries the medical procedure instruction, to determine a second medical action that corresponds to the medical procedure instruction and executed by the second medical device, and to execute the second medical action when execution requirement of the second medical action satisfies a preset requirement.

The medical device system will be described in combination with FIG.4. As shown in FIG.4, the medical device system includes two medical devices, namely medical device A and medical device B. Each medical device in the medical device system can acquire a voice signal of the user. That is, each medical device can acquire the same voice signal, but the medical device only determines the medical action to be executed by itself according to the medical procedure instruction in the voice signal. For example, the medical device A determines a medical action a according to the medical procedure instruction in the voice signal and executes the medical action a, while the medical device B determines a medical action b according to the medical procedure instruction in the voice signal and executes the medical action b.

A more specific application example is as follows. The medical device system includes a monitor and an anesthesia machine communicationally connected with each other, and both of with have an audio acquisition apparatus and a processor. The voice signal of the user can be acquired by the monitor and the anesthesia machine respectively. For example, the medical procedure instruction carried by the voice signal is an instruction to inject a certain anesthetics after BIS exceeds a certain value. The monitor analyzes and processes the medical procedure instruction, determines that the medical action that corresponds to the medical procedure instruction and required to be executed by itself is to monitor whether the BIS value reaches a certain value, and sends a triggering command to the anesthesia machine when the BIS value reaches a certain value. The anesthesia machine analyzes and processes the medical procedure instruction, determines that the medical action that corresponds to the medical procedure instruction and required to be executed by itself is to inject the anesthetic indicated by the triggering command after receiving the triggering command. The monitor and anesthesia machine execute their own medical actions to complete the anesthesia process cooperatively.

A more specific application example is as follows. The medical device system includes a monitor and a ventilator, and both of with have an audio acquisition apparatus and a processor. The voice signal of the user can be acquired by the monitor and ventilator respectively. For example, the medical procedure instruction carried by the voice signal is implementing an endotracheal intubation. The monitor analyzes and processes the medical procedure instruction, and determines that the medical action that corresponds to the medical procedure instruction and required to be executed by itself is to suspend breathing parameter monitoring. The ventilator analyzes and processes the medical procedure instruction, and determines that the medical action that corresponds to the medical procedure instruction and required to be executed by itself is to suspend ventilator ventilation. The two medical devices execute their own medical actions independently.

When multiple medical devices are communicationally interconnected with each other, the audio acquisition apparatus of the medical devices can be turned on in different ways.

If the multiple medical devices have image acquisition apparatus, one method for turning on the multiple medical devices is giving a gesture. Specifically, the first medical device includes a first audio acquisition apparatus, a first image acquisition apparatus and a first processor, the second medical device includes a second audio acquisition apparatus, a second image acquisition apparatus and a second processor. The first image acquisition apparatus is operable to acquire an image and send the image to the first processor. The first processor is operable to detect from the image the gesture which satisfies a preset requirement. If the gesture which satisfies the preset requirement is detected, the first audio acquisition apparatus is turned on by the first processor. The second image acquisition apparatus is operable to acquire an image and transmitting the image to the second processor. The second processor is operable to detect from the image the gesture which satisfies a preset requirement. If the gesture which satisfies the preset requirement is detected, the second audio acquisition apparatus is turned on. It should be noted that the gestures inputted by the user to different medical devices can be the same or different. If the acquisition perspectives of the image acquisition apparatuses of the medical devices are appropriate, the gesture given by the user can be acquired by multiple medical devices at the same time, and the user can turn on the multiple medical devices at the same time with the same gesture.

Another method is turning on the audio acquisition apparatus of one medical device, and then triggering other medical devices to turn on their audio acquisition apparatuses. Specifically, the first medical device includes a first audio acquisition apparatus, and the second medical device includes a second audio acquisition apparatus. The first medical device is operable to generate a turning-on command when the first audio acquisition apparatus is turned on and send the turning-on command to the second medical device. The second medical device is operable to turn on the second audio acquisition apparatus after receiving the turning-on command. The method for turning on the first audio acquisition apparatus can be any of the above. This method for turning on is based on the interconnection function between the medical devices. When the audio acquisition apparatus is turned on, the medical device sends the turning-on status to other medical devices communicationally connected to trigger the other medical devices to turn on their own audio acquisition apparatus.

Another method is arranging a control device in the medical device system, communicationally connecting one or more medical device with the control device, and controlling the turning-on of the audio acquisition apparatuses of the medical devices by the control device. Specifically, the first medical device and the second medical device are respectively communicationally connected with the control device. The first medical device includes a first audio acquisition apparatus, and the second medical device includes a second audio acquisition apparatus. The control device is operable to send a turning-on command to the first medical device and the second medical device after receiving an instruction to turn on the audio acquisition apparatuses. The first medical device is operable to turn on the first audio acquisition apparatus after receiving the turning-on command. The second medical device is operable to turn on the second audio acquisition apparatus after receiving the turning-on command. The method for turning on the control device can be any of the above. In this method for turning on, the medical devices in the medical device system is managed by an independent control device. In this way, only one control device needs to be provided with components such as image acquisition apparatus to receive the instruction to turn on the audio acquisition apparatuses, and each medical device in the system does not need to be provided with such components, so as to simplify the design of medical device.

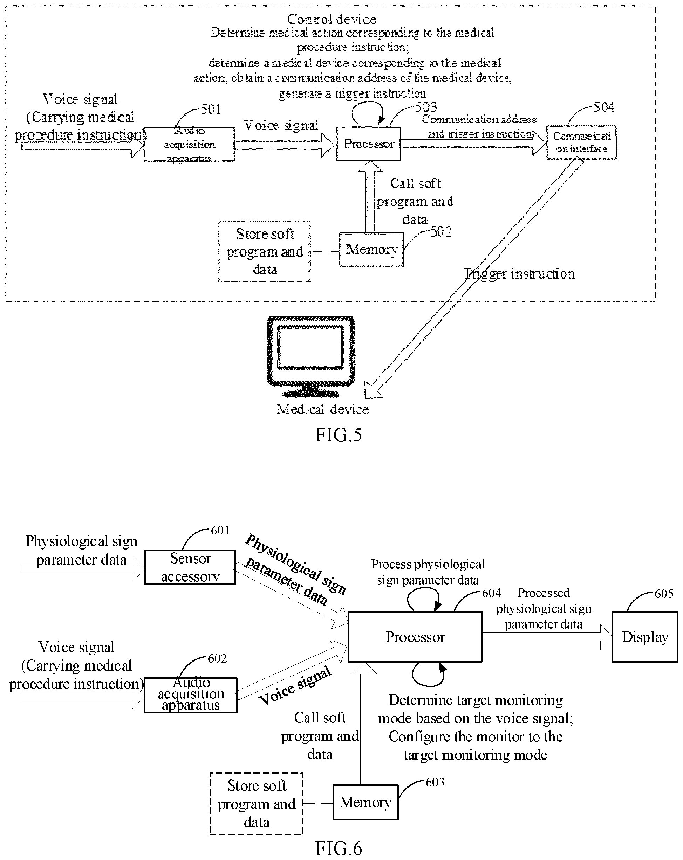

Further, in addition to turning on the audio acquisition apparatus of the medical device, the control device can also have the function of analyzing the medical procedure instructions. After the medical action is analyzed, one or more medical devices connected with the control device are triggered to execute the medical action. Specifically, as shown in FIG. 5, this disclosure provides a control device, which includes an audio acquisition apparatus 501, a memory 502, a processor 503 and a communication interface 504.

The audio acquisition apparatus 501 is operable to acquire a voice signal which carries a medical procedure instruction and is inputted by a user and to send the voice signal to the processor 503. The memory 502 is operable to store a software program and data. The processor 503 is operable to run the software program stored in the memory, call the data stored in the memory by, and at least execute the following steps of determining a medical action that corresponds to the medical procedure instruction, determining a medical device which corresponds to the medical action, obtaining a communication address of the medical device, generating a triggering command which triggers the medical device to execute the medical action, sending the communication address of the medical device and the triggering command to the communication interface. The communication interface 504 is operable to send the triggering command to the medical device according to the communication address of the medical device.

It should be noted that the control device needs to record at least a first recorded information which contains a corresponding relationship between the medical procedure instructions and the medical actions; a second recorded information which contains a corresponding relationship between the medical action and the medical device, wherein the corresponding relationship between the medical action and the medical device indicates that the medical action needs to be executed by the medical device; and a third recorded information which contains a communication address of the medical device. In this way, after extracting the medical procedure instruction, the processor of the control device can determine the medical action that corresponds to the medical procedure instruction according to the first recorded information, determine which medical device needs to execute the medical action according to the second recorded information, and determine the communication address of the medical device according to the third recorded information. Then the triggering command can be sent to the communication address through the communication interface 504 for triggering the medical device to execute the medical action. The triggering command can include the related information of the medical action, so that the medical device can execute the medical action according to the related information after receiving the triggering command.

In practical application, when executing a complex medical procedure for a patient, it is necessary to configure the interface data and workflow of the monitor in an all-round way, which can be realized by configurating the monitoring mode. This disclosure also provides a monitor, and the user can instruct the monitor to switch the monitoring mode through a voice signal.

As shown in FIG.6, the monitor specifically includes a sensor accessory 601, an audio acquisition apparatus 602, a memory 603, a processor 604 and a display 605.

The sensor accessory 601 is operable to acquire physiological parameter data of a monitored object, and to send the physiological parameter data to the processor 604.

The audio acquisition apparatus 602 is operable to acquire a voice signal which is inputted by a user and indicates to switch to a target monitoring mode, and to send the voice signal to the processor 604.

The processor 604 is operable to process the physiological parameter data, to send the processed physiological parameter data to the display 605 for displaying, to determine the target monitoring mode based on the voice signal, to determine configuration information which corresponds to the target monitoring mode, and to configure the monitor according to the configuration information.

The display 605 is operable to display the physiological parameter data which is processed by the processor 604.

Wherein, the configuration information may include monitoring interface configuration information, then the processor is operable to configure the monitor according to the configuration information through following steps. The processor is operable to control the display 605 to display the target monitoring interface that corresponds to the monitoring interface configuration information based on the monitoring interface configuration information.

Specifically, the monitoring interface configuration information represents an information containing a style and content of the generated monitoring interface. The processor can generate a monitoring interface with the certain style and content according to the monitoring interface configuration information, which can be called as the target monitoring interface.

It can be seen that on the one hand, the above monitor can acquire the physiological parameter data of the monitored object, process the physiological parameter data, such as generate the monitoring interface including the real-time value, waveform diagram and trend diagram of physiological parameters, and so on, and display the processed results to the user. On the other hand, the above monitor can also acquire a voice signal of the user, obtain the configuration information which corresponds to the monitoring mode according to the instruction to switch to a target monitoring mode in the voice signal, and configure the monitor in an all-round way according to the configuration information. The configuration information can include the configuration of the display, the configuration of the processor, the configuration of various detection accessories, etc.

In addition to configurating the monitor to a desired monitoring mode through a voice signal, the monitor can also be instructed to execute some desired medical actions through the voice signal in this monitoring mode.

Therefore, based on the above functions, the audio acquisition apparatus is also operable to acquire the voice signal which carries the medical procedure instruction that is related to the target monitoring mode and send the voice signal to the processor. The processor is also operable to extract the medical procedure instruction related to the target monitoring mode from the voice signal, determine a medical action which corresponds to the medical procedure instruction that is related to the target monitoring mode, and control execution of the medical action. It should be noted that for the description of the medical procedure instructions, please refer to the above medical procedure instructions. The medical actions which are expected by the user to be executed through the monitor are all the medical actions in the target monitoring mode.

The following describes the working process of the medical devices, such as monitors in combination with several application scenarios.

Application scenario example 1, a patient transportation scenario, which includes various types of transportation scenarios, such as an in-hospital transportation and an out-hospital transportation

During transportation, the medical staff and patient are usually in a moving state, and the external environment is relatively unstable. At this time, it is very inconvenient for the medical staff to manually operate medical device, and the manual operation is prone to misoperate. Therefore, when preparing to transport the patient, the audio acquisition apparatus of the medical device can be turned on and the voice instruction function can be activated, so that some functions of the medical device can be realized through a voice control during the transportation process, so as to greatly improve the operation accuracy and efficiency.

Firstly, the monitor is instructed by a voice signal to enter the indoor transportation mode or outdoor transportation mode. For example, the user inputs the voice signal of "indoor transportation mode", and the monitor configures itself to the indoor transportation mode in response to the voice signal. The specific configurations include but are not limited to switching a displayed interface into a large font interface, simplifying interface elements to hide unnecessary information, such as hiding the alarm limit, increasing the alarm volume, and automatically switching the monitoring parameters. The reason for automatic switching the monitoring parameters is that the parameters monitored during the transportation may be different from those during the non-transportation scenarios. For example, invasive blood pressure monitoring and airway monitoring of respiratory rate will be carried out in the inpatient ward, and non-invasive blood pressure monitoring and impedance monitoring of respiratory rate will be carried out during transportation. It should be noted that the transportation mode can be specifically called as an indoor transportation mode, which corresponds to the indoor transportation scenario. The user can also control the monitor to enter the outdoor transportation mode by a voice signal. For example, if an outdoor environment of strong light is experienced during the transportation, the user inputs the voice signal "outdoor transportation mode", and the monitor configures itself to the outdoor transportation mode in response to the voice signal. The specific configurations include but are not limited to adopting an outdoor display mode. The outdoor display mode is displayed in a high contrast. Generally, a white or orange high contrast is adopted to avoid the difficulty of viewing the display screen caused by too strong outdoor sunlight.

In the transportation mode, the monitor is instructed to execute a noninvasive blood pressure measurement by a voice signal. Specifically, when it is necessary to know the blood pressure of the patient, the medical staff control the monitor to start a single NIBP measurement through the voice signal of "NIBP measurement". If it is necessary to conduct a regular noninvasive blood pressure measurement, the monitor is controlled to start an NIBP measurement every 5 minutes through the voice signal of "5 minutes NIBP measurement".

In the transportation mode, the monitor is instructed to execute an invasive blood pressure zero calibration. Specifically, the turbulence during the transportation may lead to inaccurate monitoring of invasive blood pressure (IBP), so it is necessary to execute the zero calibration on the invasive blood pressure monitoring. The zero calibration of the invasive blood pressure channel can be the zero calibration of one channel, or the zero calibration of multiple channels at the same time, or the zero calibration of all the channels at the same time. Specifically, for the zero calibration of a specific IBP channel, such as an arterial pressure (Art) channel or central venous pressure (CVP) channel, after preparing for the zero calibration, a voice instruction of "Art zero calibration" or "CVP zero calibration " can activate the zero calibration. And the blood pressure measurement can be recovered after the zero calibration. Specifically, for the zero calibration of multiple channels or all channels, such as Art and CVP channels and so on, after all preparing for the zero calibration, a voice instruction of" zero calibration of Art and CVP two channels" or " zero calibration of all IBP" can activate the zero calibration. And the blood pressure measurement can be recovered after the zero calibration. In the whole process, both hands of the medical staff can be put into the treatment of the patient without manually operating the monitor. At the same time, the hands wearing sterile gloves can be prevented from being polluted by contacting the monitor.

In the transportation mode, the monitor is instructed to enter an endotracheal intubation mode. Specifically, before the endotracheal intubation, the medical staff usually have sterile gloves on both hands, which is not suitable for contacting the monitor at this time. Therefore, the monitor can be instructed to enter the endotracheal intubation mode through a voice signal of "enter intubation mode". In this mode, the monitor can block the parameter monitoring alarms related to the respiratory system, such as the respiratory rate, end breath carbon dioxide and respiratory mechanics monitoring.

In the transportation mode, the monitor is instructed to enter a rescue mode. Specifically, if the patient is in a critical condition and needs to be rescued, the medical staff mainly focus on the patient and cannot manually operate the monitor to switch it to the rescue mode in time. Therefore, the monitor can be instructed to switch to the rescue mode through a voice signal of "enter rescue mode".

In the transportation mode, the monitor is instructed execute a 12-lead ECG analysis. Specifically, patients with chest pain needs ECG analysis in time to determine whether a cardiac intervention is needed. Therefore, the medical staff manually connect the ECG electrode for the 12-lead ECG analysis to the patient. At this time, the main operation of the medical staff is focused on the patient, and then the monitor can be instructed to enter the special interface for the 12-lead analysis through a voice signal of "12-lead analysis", so as to start the 12-lead analysis and obtain the analysis results. Then, the analysis results can be sent to the devices in the emergency center through the communication network via a voice signal of "send 12-lead report". The experts of the emergency center will determine whether the patient needs the cardiac bypass surgery. If necessary, an ambulance will be arranged to go to the nearest hospital capable of performing the bypass surgery.

In the transportation mode, the related information of operation events can be recorded by a voice signal. Specifically, emergencies such as cardiac arrest and so on, may occur during the transportation of patients. At this time, due to the limited number of first-aid personnel, it may not be possible to arrange a medical staff to record the first-aid process. Thus, the medical staff can dictate the operation steps while giving first aid to the patient. The monitor converts the voice into words to form an operation event and adds a time mark to the operation event to form a first aid event record table.

In the transportation mode, the infusion pump can be turned on or off by a voice signal. Specifically, if the infusion pump is used for administration during the transportation, there may be a need to stop the administration or suspend a certain drug administration. At this time, the position of the infusion pump is lower than that of the medical staff, and the operation is inconvenient. Therefore, the medical staff can use a voice signal to control the operation of the infusion pump, such as turning on or off one or more infusion pumps, stopping the injection of a drug, and injecting a drug at a certain speed. The voice signal can include for example, "stop dopamine injection" and " inject dopamine with XX ml/min".

In the transportation mode, the monitor is instructed to communicate with other devices by a voice signal. Specifically, some patients who can move within a certain range will wear wearable mobile devices for monitoring, such as a remote-control device, a transportation monitor, a wearable measurement module, etc. When they leave the hospital beds, the medical staff controls the designated mobile device to pair with the designated bedside monitor through an input voice signal, so that the medical staff can watch the vital signs monitoring of nearby active patients from the large screen of the bedside monitor of the patient in the ward. This technology can also be applied to patients transported from place A to place B. The medical staff can wirelessly pair the mobile device transported with the patient to place B with the existing monitor at place B through a voice control.

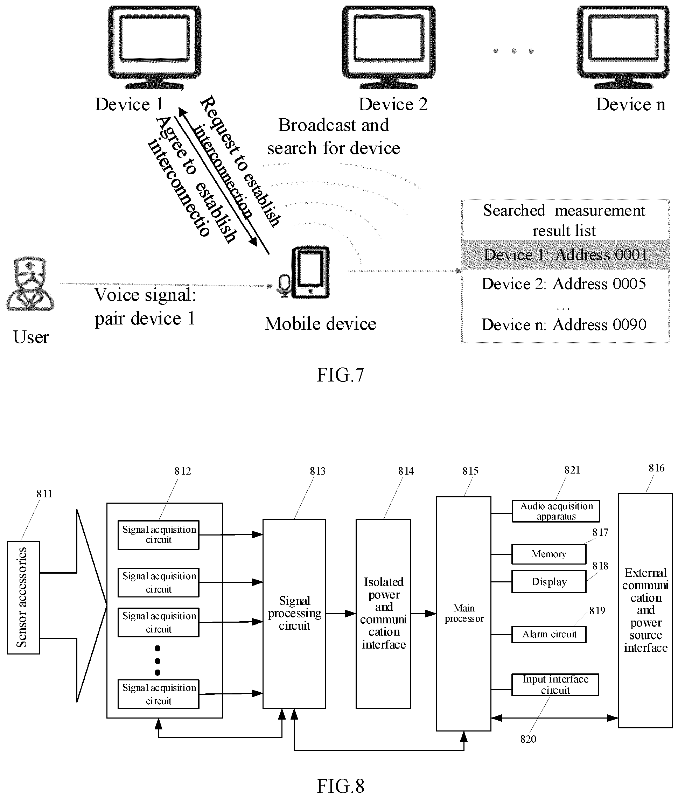

Referring FIG.7, an example of pairing a mobile device with a monitor is shown. The mobile device is a monitoring device which is transported to a certain place with the patient. When its voice acquisition apparatus is turned on, the mobile device can acquire the voice signal of "pair device 1" sent by the user. Further, the mobile device sends a device search message to obtain a list of searched devices. The mobile device determines device 1 in the device list and initiates a communication request with device 1 to establish an interconnection according to the address of device 1. Device 1 agrees to establish the interconnection, then the interconnection between the device 1 and the mobile device is completed. The voice signal of "device 1" which is operable to distinguish the devices can be a device name, a device associated hospital bed number, device number, etc., which can identify the equipment identity.

It should be noted that the above voice control technology is not only applicable to the monitor, but also can be extended to a variety of medical devices which may be used in the transportation process, such as a defibrillation monitor, a ventilator, an infusion pump, an oxygen supply device, an extracorporeal membrane oxygenation (ECMO) and other monitoring and treatment devices.

Application scenario example 2, a perioperative scenario, which includes an induction period, a maintenance period and a recovery period.

During the perioperative period, the surgeon is mainly responsible for the operation of the patient, and the anesthesiologist is responsible for the management of the medical devices, such as a monitor, anesthesia machine, ventilator and infusion pump. In order to create a suitable operation position for the surgeon for different types of operations, the anesthesiologist always spends a certain time adjusting the positions of the medical devices around the operating table in combination with the anesthesia mode, the body position of the patient, the position of the operation, the position of the operation device and other factors. Once the positions of these medical devices are determined, they generally will not move during the operation, which may cause inconvenience for anesthesiologist to operate the medical devices. Therefore, anesthesiologist can use the voice signal to control the medical devices, which can not only improve the convenience of the device operation, but also make the standing position of anesthesiologist more flexible during operation.

In the induction phase, anesthesiologist usually give drugs through a mask. At this time, anesthesiologist mainly stands near the head of the patient, and gives the patient some drugs through an infusion pump to a push injection, and then observes the monitored parameters of the monitor to determine the anesthesia state and anesthesia depth of the patient and confirm whether the dosage of drugs is appropriate. At this stage, the anesthesiologist is standing beside the patient for anesthetizing the patient, and there may be a certain distance from these medical devices, so it is inconvenient for the anesthesiologist to operate these medical devices. Therefore, the medical device can be controlled by the voice signal to realize the following functions.

First, the voice signal of "enter induction period monitoring" is inputted to the monitor to control the monitor to enter a surgery induction period monitoring mode. In this mode, the monitor provides, but is not limited to only provide, a monitoring interface suitable for the induction period, which includes common monitored parameter values and trend charts in the induction period. In addition, if the monitor determines that the patient has not experienced an invasive blood pressure monitoring, it starts the noninvasive blood pressure monitoring.

Then, the anesthesia machine is controlled by a voice signal to enable the patient to inhale an appropriate amount of anesthetic gas. For example, the voice signal is "anesthesia machine with 4% concentration sevoflurane", and the anesthesia machine is controlled to send sevoflurane gas with a concentration of 4% to the patient.

Then, one or more infusion pumps are controlled by a voice signal to inject intravenous anesthetic drugs into the patient. For example, the voice signal is "propofol 2.0mg/kg multiply 60kg", and the infusion pump provided with propofol is controlled to inject 120mg propofol into the patient according to the instruction of the voice signal.

Then, after determining that the patient enters the anesthesia state, the artery of the patient is opened and catheterized for IBP monitoring. At this time, the voice signal of "IBP zero calibration" can be used to control the monitor for IBP zero calibration.

Then, the voice signal of "start monitor muscle relaxation TOF mode" is inputted to enable the monitor to select the muscle relaxation working mode as "TOF mode" and start the muscle relaxation monitoring. In addition, the infusion pump can also be controlled to inject drugs which are related to the muscle relaxation monitoring into the patient. For example, the anesthesiologist sends a voice signal of "pump muscle relaxation drug midazolam 4mg" to control the infusion pump provided with midazolam to inject the midazolam which is related to the muscle relaxation into the patient. TOF (Train Of Four) mode is a specific stimulation mode of muscle relaxation. The stimulation mode of muscle relaxation started by the voice signal can also include others, such as a single stimulation mode of muscle relaxation, a forced stimulation mode of muscle relaxation, a forced stimulation mode after muscle relaxation, a double burst stimulation mode of muscle relaxation, etc.

During the maintenance period, anesthesiologist need to maintain the stability of the vital signs of the patient and ensure that the state of the patient satisfies the operation requirement. Therefore, he/she needs to continue paying attention to the changes of the vital signs of the patient and give drugs in time in some cases, such as operating the infusion pump for administration, operating the anesthesia machine to give inhaled anesthetic gas and oxygen, etc. At this time, anesthesiologist usually stands or sits next to the anesthesia machine, which may be far away from the monitor, infusion pump and other devices, and the operation is not easy. Therefore, they can use the voice signal input to control the medical device to realize the following functions.

When the patient comes to the surgery state, the anesthesiologist can control the anesthesia machine to stop the drug administration through the voice signal. When the anesthesia state of the patient gradually becomes light, the drug administration of the anesthesia machine can be controlled to start through the voice signal. When the drug injection is needed, the anesthesiologist also can control the infusion pump by a voice, for example, control a certain infusion pump to input the dosage of 0.2ug by a voice signal.

During the resuscitation stage, the patient is sent to the resuscitation room, and the mobile device, such as the transportation monitor also follows the patient to the resuscitation room. At this time, the medical staff can control the mobile device to send the patient data to the designated monitor in the resuscitation room through a voice signal to ensure the integrity of the patient data throughout the perioperative period. The voice signal can include but is not limited to " transfer patient", and the patient data includes but not limited to patient information, monitored values of vital sign parameters, waveforms of vital sign parameters, alarm information, resuscitation score results, etc.

Application scenario example 2, a bedside monitoring mode for in-hospital patent, which is especially the scenario of hemodynamic monitoring on the circulatory system, and can specifically include an intensive monitoring mode, a sub-intensive monitoring mode and a general ward monitoring mode.

During the monitoring and treatment of the vital signs of the patient, the medical staff mainly carry out a series of nursing and treatment operations on the patient. When the medical staff are busy or understaffed, they are not convenient to manually operate the medical device, and then the medical device can be controlled through the voice signal to liberate the hands of the medical staff, which improves the convenience of the use of the medical device and reduces the time for learning the use of the device. Clinically, the functions of the medical devices suitable for controlling by a voice signal include the following aspects.

Controlling ECG measurement function by a voice signal specifically includes, but is not limited to controlling displaying which ECG waveforms (such as II lead ECG waveform, I lead ECG waveform, V lead ECG waveform, etc.) by a voice signal; adjusting a filtering mode by a voice signal, such as turning on a ST analysis switch when analyzing ST segment in ECG and configurating the filtering mode to an ST mode and employing an operation mode during operation; marking the pacemaker with an activated state for the patient with cardiac pacing; and turning on an arrhythmia analysis switch for the patient with arrhythmia.

Controlling noninvasive blood pressure measurement by a voice signal specifically includes, but is not limited to adjusting an initial inflation pressure value by a voice signal; selecting one measurement mode from a plurality of measurement modes which include a manual mode, an interval mode, a continuous mode, a sequence mode and so on by a voice signal and selecting an interval duration by a voice signal in the interval mode; starting NIBP measurement by a voice signal; starting one time of manual measurement by a voice signal during the interval measurement; opening NIBP measurement list to display the measurement results over a period of time by a voice signal; and configurating the alarm limit of noninvasive blood pressure according to the situation of the patient by a voice signal. For example, the voice signal of "NIBP alarm limit" can open the alarm limit configuration of corresponding parameters, and then the alarm limit can be configurated through a voice signal. Of course, this alarm limit configuration is not limited to the blood pressure parameters, and the alarm limit of other physiological parameters can also be configurated by voice signals.

Controlling invasive blood pressure measurement by a voice signal specifically includes but is not limited to regarding to a specific IBP measurement such as Art, starting Art zero calibration after preparing for zero calibration by a voice signal; and regarding to multiple IBP measurement such as Art and CVP, starting multiple-channel zero calibration after all preparing for zero calibration by a voice signal.

Controlling cardiac displacement measurement by a voice signal specifically includes but is not limited to inputting height and weight of the patient by a voice signal for the device to calculate a body surface area, which can be used for cardiac displacement or continuous cardiac displacement measurement. For example, the voice signal input to the medical device is "height 175 cm, weight 58 kg". The medical device displays the calculation result of the body surface area as "1.706 square meters" on the interface and gives a voice broadcast of" 1.706 square meters". Alternatively, the medical device can also calculate the body mass index (BMI) of the patient, and then display it on the interface and give a voice broadcast.

Controlling a playing process of hemodynamic physiology pattern by a voice signal specifically includes, but is not limited to controlling a play speed of the hemodynamic physiology pattern (such as playing the hemodynamic physiology pattern in a slow speed which enable the doctor to see the parameter measurement values of various parts clearly), by a voice signal such as "playing at half speed" or reducing a half speed"; or freezing the hemodynamic physiology pattern by a voice signal, such as "freeze physiology pattern ".

Controlling a passive leg lifting test or capacity load test by a voice signal specifically includes but is not limited to opening an observation window of the passive leg lifting test by a voice signal; controlling the passive leg lifting test to start, activating the functions and marking the baseline by a voice signal; and controlling the passive leg lifting test to end by a voice signal. The operating process on the patient by the medical staff to perform the leg lifting test includes following steps. First, let the patient to stand slightly with his/her upper body, and then quickly flatten his/her upper body and left his/her legs, such that the blood in the patient can quickly return to the heart of the patient. Then let the patient to restore the normal bed state for a period of time to observe the changes of hemodynamic parameters.

Controlling a sepsis screening and treatment process by a voice signal can be as follows. Firstly, a sepsis related organ failure assessment function on the monitor is started for sepsis screening by a voice signal of "SOFA score". Then the operator takes the biochemical examination results of the patient and read them out successively according to the SOFA scoring items. After receiving these voice information, the monitor scores them according to the scoring requirement and gives treatment suggestions according to the screening rule. For example, the biochemical examination results included in the voice signal are "oxygen partial pressure 410, platelet 147, bilirubin 2.1, average arterial pressure 72, Glasgow coma score 13, blood creatinine 1.3 and urine volume 470". After receiving these voice information, the monitor will score them according to the scoring requirement, such as "0 for oxygen partial pressure 410, 1 for platelet 147, 2 for bilirubin 2.1, 0 for average arterial pressure 72, 1 for Glasgow coma score 13, 1 for blood creatinine 1.3, and 3 for urine volume 470", and then accumulate the scores to obtain a total score of 8. Then, according to the screening rule, the monitor concludes that the patient satisfies the sepsis standard and suggests a sepsis treatment.

If the patient needs a sepsis treatment, the treatment measures can also be recorded by voice. After the monitor adds the treatment measures after the occurrence time, a treatment event is generated and stored in the patient data. For example, the user inputs the voice signal "monitor patient blood glucose status" to the monitor, and the monitor adds the system time "2019-1-3" to get a treatment event "2019-1-3 monitor patient blood glucose status". It should be noted that controlling the scoring function by a voice signal is not limited to this, but also can be applied to Glasgow coma score, resuscitation score, pain score, etc.

It should be noted that the above application scenarios are only examples, and the application of the medical devices is not limited to this. Through controlling the functions of the medical device by a voice signal, the manual control steps of users can be simplified and the convenience of controlling the device can be improved.

The medical device can be specifically a monitor, and a specific example of which is shown in FIG.8. FIG. 8 provides a system framework diagram of a parameter processing module in a multi-parameter monitor.

The multi-parameter monitor has an independent housing, whose panel is provided with a sensor interface zone which is integrated therein multiple sensor interfaces for connecting with various external physiological parameter sensor accessories 811. The housing panel also includes a small IxD display zone, a display 818, an input interface circuit 820 and an alarm circuit 819 (such as, a LED alarm zone). A parameter processing module is used as an external communication and power source interface for communicating with a main unit and taking power from the main unit. The parameter processing module also supports a build-out parameter module, can form a plug-in monitor main unit by means of inserting the parameter module, can be used as part of the monitor, or can be connected to the main unit via a cable, with the build-out parameter module being used as an external accessory of the monitor. In addition, the multi-parameter monitor includes a memory 817 for storing computer programs and various data generated during the related monitoring process.

The internal circuit of the parameter processing module is disposed inside the housing. As shown in FIG.8, the internal circuit includes a signal acquisition circuit 812 which corresponds to at least two physiological parameters, a front-end signal processing circuit 813 and a main processor 815.

The main processor 815 can execute the processing functions described in any embodiments above.

The signal acquisition circuit 812 may be selected from an electrocardiogram circuit, a respiration circuit, a body temperature circuit, a blood oxygen saturation circuit, a non-invasive blood pressure circuit, an invasive blood pressure circuit, etc. These signal acquisition circuits 812 are electrically connected with corresponding sensor interfaces for electrically connecting to sensor accessories 811 which correspond to different physiological parameters. An output terminal of the signal acquisition circuit 812 is coupled to the front-end signal processing circuit 813 whose communication terminal is further coupled to the main processor 15. The main processor 815 is electrically connected with the external communication and power interface.

Various general circuits and modules known in the prior art can be operable to realize the signal acquisition circuit 812 for various physiological parameters. The front-end signal processing circuit can be configured to complete the sampling and analog-to-digital conversion of output signals of the signal acquisition circuit 812, and output control signals to control the physiological parameter measurement process. The physiological parameters include but are not limited to parameters of electrocardiogram, respiration, body temperature, blood oxygen, non-invasive blood pressure, and invasive blood pressure.

The front-end signal processing circuit can be realized by a single-chip microcomputer or other semiconductor devices, or ASIC or FPGA. The front-end signal processing circuit can be powered by an isolated power supply. After a simple processing and packaging, the sampled data can be sent to the main processor through an isolated communication interface. For example, the front-end signal processing circuit can be coupled to the main processor 815 through the isolated power and communication interface 814.

The audio acquisition apparatus 821 is connected to the processor 815. The audio acquisition apparatus 821 may be specifically a microphone or the like which is operable to acquire voice signals.

Supplying electrical power to the front-end signal processing circuit through the isolated power supply has a function of isolating the patient from the power supply device through isolating the DC/DC power supply via a transformer. In such a way, the application part is floating through the isolation transformer, such that the leakage current passing through the patient is small enough, and bad influences on boards and devices of intermediate circuits, such as main control board (guaranteed by creepage distance and electrical clearance), due to voltage or energy generated during a defibrillation or electric knife application, can be prevented.

The main processor completes the calculation of physiological parameters and sends calculation results and waveforms of the physiological parameter to the main unit (such as, a main unit with a display, PC, a central station, etc.) through the external communication and power interface. The external communication and power source interface 816 may be one or a combination of local area network interfaces composed of Ethernet, Token Ring, Token Bus, and optical fiber distributed data interface (FDDI) as the backbone of these three networks, may also be one or a combination of wireless interfaces such as infrared, Bluetooth, WIFI, and WMTS communication, or may also be one or a combination of wired data connection interfaces such as RS232 and USB.

The external communication and power source interface 816 may also be one or a combination of the wireless data transmission interface and the wired data transmission interface. The main unit may be any computer device such as a main unit of the monitor, an electrocardiograph, an ultrasonic diagnosis instrument, a computer, etc. A monitor can be formed by means of installing them with matching software. The main unit may also be a communication device, such as a mobile phone, and the parameter processing module sends data to a mobile phone that supports Bluetooth communication via a Bluetooth interface to realize remote data transmission.

It should be noted that each embodiment in this disclosure is described in a progressive manner. Each embodiment focuses on the differences from the other embodiments. The same and similar parts of each embodiment can be referred to each other.

It should also be noted that in this disclosure, relational terms such as first and second are only operable to distinguish one entity or operation from another entity or operation, and do not necessarily require or imply any such actual relationship or order between these entities or operation. In addition, the terms "includes", "having", and any variations thereof are intended to cover non-exclusive inclusion. For example, a process, a method, a system, a product, or a device that includes a series of steps or units is not limited to the listed steps or units, but optionally further includes unlisted steps or units, or optionally further includes other steps or units inherent in these procedures, methods, or devices. Without further restrictions, the elements defined by the statement "includes a..." do not exclude the existence of other same elements in the process, method, article or device including the above elements.

The above description of the disclosed embodiments enables those skilled in the art to realize or use this disclosure. Various modifications to these embodiments will be apparent to those skilled in the art, and the general principles defined herein can be implemented in other embodiments without departing from the spirit or scope of this disclosure. Therefore, this disclosure will not be limited to these embodiments shown herein but will conform to the widest scope consistent with the principles and novel features disclosed herein.

## Claims

1. A medical device, **characterized in that**, comprising:
an audio acquisition apparatus, which is operable to acquire a voice signal and to send the voice signal to a processor; wherein the signal carries a medical procedure instruction and is inputted by a user, the medical procedure instruction is operable to instruct the medical device to execute a preset medical procedure, wherein the medical procedure comprises one or more preset medical actions, and there is a preset corresponding relationship between the medical procedure instruction and the medical action(s); and
the processor, which is operable to determine the medical action(s) that corresponds to the medical procedure instruction, and to control execution of the medical action(s), after receiving the voice signal.

2. The medical device according to claim 1, **characterized in that**, the processor is further operable to receive an instruction to turn on the audio acquisition apparatus, and to turn on the audio acquisition apparatus after receiving the instruction to turn on the audio acquisition apparatus.

3. The medical device according to claim 2, **characterized in that**, the medical device further comprises at least one of: a physical key, a touch-sensitive display, a photoelectric sensor, a biological characteristic acquisition apparatus, an image acquisition apparatus, and a communication interface;
wherein the processor is further operable to receive the instruction to turn on the audio acquisition apparatus through any one of following ways:
when receiving a triggering command, the physical key generates the instruction to turn on the audio acquisition apparatus and sends the instruction to turn on the audio acquisition apparatus to the processor;
when receiving a preset gesture trace, the touch-sensitive display generates the instruction to turn on the audio acquisition apparatus and sends the instruction to turn on the audio acquisition apparatus to the processor;
when detecting a change of light which satisfies a preset requirement, the photoelectric sensor generates the instruction to turn on the audio acquisition apparatus and sends the instruction to turn on the audio acquisition apparatus to the processor;
when acquiring biological characteristic information of the user, the biological characteristic acquisition apparatus sends the biological characteristic information to the processor, then the processor is further operable to determine that the processor receives the instruction to turn on the audio acquisition apparatus when the biological characteristic information is same as prestored biological characteristic information
when acquiring a barcode image, the image acquisition apparatus sends the barcode image to the processor, then the processor is further operable to extract identity information from the barcode image, and to determine that the processor receives the instruction to turn on the audio acquisition apparatus when the extracted identity information is same as prestored identity information;
when acquiring an image, the image acquisition apparatus sends the image to the processor, then the processor is further operable to detect from the image a gesture that satisfies a preset requirement, and to determine that the processor receives the instruction to turn on the audio acquisition apparatus when the gesture that satisfies the preset requirement is detected; or
the processor is further operable to receive the instruction to turn on the audio acquisition apparatus which is sent by other devices through the communication interface.

4. The medical device according to claim 1, **characterized in that**, in order to determine the medical action(s) that corresponds to the medical procedure instruction, the processor is specifically operable to:
search for a target medical procedure instruction in a preset instruction database, wherein the target medical procedure instruction has a semantic similarity to the medical procedure instruction that satisfies a preset similarity threshold; and
determine the medical action that corresponds to the target medical procedure instruction in a preset medical action database.

5. The medical device according to claim 4, **characterized in that**, in order to search for the target medical procedure instruction that has the semantic similarity satisfying the preset similarity threshold, the processor is specifically operable to search for a medical procedure instruction having a semantic similarity to the medical procedure instruction that satisfies a first preset similarity threshold;
if found, the processor is specifically operable to determine the medical procedure instruction found as the target medical procedure instruction;
if not found, the processor is specifically operable to search for a medical procedure instruction having a semantic similarity to the medical procedure instruction that satisfies a second preset similarity threshold, to output the medical procedure instruction found, to receive a command for the user to select a medical procedure instruction, and to determine the medical procedure instruction selected by the user as the target medical procedure instruction.

6. The medical device according to claim 1, **characterized in that**, when there are a plurality of medical actions and the plurality of medical actions are executed by the medical device; in order to control execution of the medical actions, the processor is specifically operable to determine an execution sequence of each medical action, and to control the execution of each medical action according to the execution sequence.

7. The medical device according to claim 1, **characterized in that**, when there are a plurality of medical actions, and the plurality of medical actions include a medical action which is to be executed by another medical device;
in order to control execution of the medical actions, the processor is specifically operable to determine an execution sequence of each medical action, to determine a corresponding medical device for executing each medical action, and to trigger each medical device to execute corresponding medical action according to the execution sequence.

8. The medical device according to claim 1, **characterized in that**, the processor is operable to acquire any voice signal which carries any medical procedure instruction through the audio acquisition apparatus, to record the medical action(s) executed by the medical device within a preset time before and after an input time point of the any voice signal, and to establish a corresponding relationship between the any medical procedure instruction and the recorded medical action(s), in a self-learning mode.

9. The medical device according to claim 1, **characterized in that**, the medical device further comprises one or more of: an information output apparatus, a physiological sign detection accessory, and a drug injection component;
wherein the medical action(s) comprises one or more of following types: a medical action that triggers the information output apparatus to output related content of the medical procedure, a medical action that triggers the physiological sign detection accessory to implement a measurement, and a medical action that triggers the drug injection component to inject a drug.

10. The medical device according to claim 1, **characterized in that**, the medical device is the infusion pump, and the medical procedure instruction is an instruction for a target infusion pump to inject a target drug;
in order to determine the medical action that corresponds to the medical procedure instruction, and to control execution of the medical action, after receiving the voice signal, the processor is specifically operable to:
determine whether the infusion pump is the target infusion pump after receiving the voice signal, which carries the instruction for a target infusion pump to inject a target drug; and
determine a drug injection action that corresponds to the instruction for a target infusion pump to inject a target drug and control execution of the drug injection action, when the infusion pump is the target infusion pump.

11. The medical device according to claim 10, **characterized in that**, an instruction to inject a target drug includes one or more of: an instruction to stop injecting a target drug, an instruction to start injecting a target drug, an instruction to inject a target drug according to a target parameter.

12. The medical device according to claim 1, **characterized in that**, the medical device is the anesthesia machine, and the medical procedure instruction is an instruction to inject a target anesthetic;
in order to determine the medical action that corresponds to the medical procedure instruction, and to control execution of the medical action, after receiving the voice signal, the processor is specifically operable to:
determine a drug injection action that corresponds to the instruction to inject a target anesthetic and control execution of the drug injection action, after receiving the voice signal.

13. The medical device according to claim 1, **characterized in that**, the medical device is the ventilator, and the medical procedure instruction is an instruction to configure a target working mode or an instruction to configure a target working parameter;
in order to determine the medical action that corresponds to the medical procedure instruction, and to control execution of the medical action, after receiving the voice signal, the processor is specifically operable to:
determine a mode configuration action that corresponds to the instruction to configure a target working mode and execute the mode configuration action, after receiving the voice signal; or
determine a parameter configuration action that corresponds to the instruction to configure a target working parameter, and execute the parameter configuration action, after receiving the voice signal.

14. A medical device system, **characterized in that**, comprising a first medical device and a second medical device, wherein
the first medical device is operable to acquire a voice signal which carries a medical procedure instruction, to determine a first medical action that corresponds to the medical procedure instruction and executed by the first medical device, and to execute the first medical action when an execution requirement of the first medical action satisfies a first preset requirement; and
the second medical device is operable to acquire the same voice signal which carries the medical procedure instruction, to determine a second medical action that corresponds to the medical procedure instruction and executed by the second medical device, and to execute the second medical action when an execution requirement of the second medical action satisfies a second preset requirement.

15. The medical device system according to claim 14, **characterized in that**, the first medical device comprises a first audio acquisition apparatus, a first image acquisition apparatus and a first processor, the second medical device comprises a second audio acquisition apparatus, a second image acquisition apparatus and a second processor; wherein
the first image acquisition apparatus is operable to acquire a first image and send the first image to the first processor;
the first processor is operable to detect from the first image a gesture which satisfies a first preset requirement, and to turn on the first audio acquisition apparatus if the gesture which satisfies the first preset requirement is detected;
the first audio acquisition apparatus is operable to acquire the voice signal which carries the medical procedure instruction;
the second image acquisition apparatus is operable to acquire a second image and send the second image to the second processor;
the second processor is operable to detect a gesture which satisfies a second preset requirement from the second image, and to turn on the second audio acquisition apparatus if the gesture which satisfies the second preset requirement is detected; and
the second audio acquisition apparatus is operable to acquire the voice signal which carries the medical procedure instruction.

16. The medical device system according to claim 14, **characterized in that**, the first medical device and the second medical device are communicationally connected, wherein the first medical device comprises a first audio acquisition apparatus and the second medical device comprises a second audio acquisition apparatus;
the first medical device is operable to generate a turning-on command when the first audio acquisition apparatus is turned on, and to send the turning-on command to the second medical device;
the second medical device is operable to turn on the second audio acquisition apparatus after receiving the turning-on command.

17. The medical device system according to claim 14, **characterized in that**, the medical device system further comprises a control device communicationally connecting the first medical device and the second medical device;
wherein the first medical device comprises a first audio acquisition apparatus and the second medical device comprises a second audio acquisition apparatus;
the control device is operation to send a turning-on command to the first medical device and the second medical device after receiving an instruction to turn on an audio acquisition apparatus;
the first medical device is operable to turn on the first audio acquisition apparatus after receiving the turning-on command; and
the second medical device is operable to turn on the second audio acquisition apparatus after receiving the turning-on command.

18. A control device, **characterized in that**, the control device is communicatively connected with at least one medical device, wherein the control device comprises an audio acquisition apparatus, a communication interface and a processor;
wherein the audio acquisition apparatus is operable to acquire a voice signal, which carries a medical procedure instruction and is inputted by a user, and to send the voice signal to the processor;
the processor is operable to determine a medical action that corresponds to the medical procedure instruction, to determine a medical device that corresponds to the medical action, to obtain a communication address of the medical device, to generate a triggering command which triggers the medical device to execute the medical action, and to send the communication address of the medical device and the triggering command to the communication interface; and
the communication interface is operable to send the triggering command to the medical device according to the communication address of the medical device.

19. A monitor, **characterized in that**, comprising a sensor accessory, an audio acquisition apparatus, a processor and a display;
wherein the sensor accessory is operable to acquire physiological parameter data of a monitored object, and to send the physiological parameter data to the processor;
the audio acquisition apparatus is operable to acquire a voice signal which is inputted by a user and indicates to switch to a target monitoring mode, and to send the voice signal to the processor;
the processor is operable to process the physiological parameter data, to send the processed physiological parameter data to the display for displaying, to determine the target monitoring mode based on the voice signal, to determine configuration information that corresponds to the target monitoring mode, and to configure the monitor according to the configuration information; and
the display is operable to display the physiological parameter data which is processed by the processor.

20. The monitor according to claim 19, **characterized in that**, the configuration information comprises monitoring interface configuration information; and
in order to configure the monitor according to the configuration information, the processor is specifically operable to:
control the display to display a target monitoring interface that corresponds to the monitoring interface configuration information, based on the monitoring interface configuration information.

21. The monitor according to claim 19, **characterized in that**, the audio acquisition apparatus is further operable to acquire a voice signal which carries a medical procedure instruction that is related to the target monitoring mode, and to send the voice signal to the processor;
the processor is further operable to extract the medical procedure instruction that is related to the target monitoring mode from the voice signal, to determine a medical action which corresponds to the medical procedure instruction that is related to the target monitoring mode, and to control execution of the medical action.

22. The monitor according to claim 19 or 21, **characterized in that**, the target monitoring mode is an indoor transportation mode or an outdoor transportation mode;
the medical procedure instruction that is related to the indoor transportation mode or the outdoor transportation mode, comprises one or more of:
an instruction to execute a blood pressure measurement by using automated noninvasive manometry, an instruction to implement a blood pressure measurement by using automated noninvasive manometry periodically, an instruction to implement a zero calibration for one or more invasive blood pressure channels, an instruction to implement a zero calibration for all invasive blood pressure channels, an instruction to enter an intubation mode, an instruction to enter a rescue mode, an instruction to implement 12-lead analysis, an instruction to send 12-lead report, an instruction to communicatively pair a target mobile device and the monitor.

23. The monitor according to claim 19 or 21, **characterized in that**, the target monitoring mode is a surgery induction period monitoring mode or a surgery resuscitation period monitoring mode;
wherein the medical procedure instruction that is related to the surgery induction period monitoring mode comprises one or more of: an instruction to implement a noninvasive blood pressure measurement, an instruction to implement a zero calibration for an invasive blood pressure channel, an instruction to start a muscle relaxation stimulation mode; and
the medical procedure instruction that is related to the surgery resuscitation period monitoring mode comprises one or more of: an instruction to implement a noninvasive blood pressure measurement, an instruction to implement a zero calibration for an invasive blood pressure channel, an instruction to send data related to the monitored object to a target monitor.

24. The monitor according to claim 19 or 21, **characterized in that**, the target monitoring mode is a bedside monitoring mode;
wherein the medical procedure instruction that is related to the bedside monitoring mode comprises one or more of:
an instruction to implement an ECG measurement, an instruction to implement a noninvasive blood pressure measurement, an instruction to implement a zero calibration for a blood pressure channel, an instruction to measure a cardiac discharge, an instruction to input a physiological index value of the monitored object, an instruction to control a hemodynamic physiology pattern, an instruction to configure a test parameter of a passive leg lifting test, an instruction to implement a sepsis screening and treatment.

25. The monitor according to claim 24, **characterized in that**:
the instruction to implement an ECG measurement, comprises one or more of:
an instruction to display a target ECG waveform, an instruction to adjust a filtering mode, an instruction to mark a pacemaker as an activated state, and an instruction to start an arrhythmia analysis;
the instruction to implement a noninvasive blood pressure measurement, comprises one or more of: an instruction to adjust an initial inflation pressure value, an instruction to select a target measurement mode, an instruction to implement a blood pressure measurement by using automated noninvasive manometry, an instruction to open a measurement result list, and an instruction to configure an alarm limit of a physiological parameter;
the instruction to implement a zero calibration for a blood pressure channel, comprises one or more of: an instruction to implement a zero calibration for an arterial pressure channel, an instruction to implement a zero calibration for two channels of an arterial pressure and a central venous pressure, and an instruction to implement a zero calibration for all invasive blood pressure channels;
the instruction to control a hemodynamic physiology pattern, comprises one or more of: an instruction to freeze the hemodynamic physiology pattern, an instruction to play the hemodynamic physiology pattern according to a target speed;
the instruction to configure a test parameter of a passive leg lifting test, comprises one or more of: an instruction to open an observation window of the passive leg lifting test, an instruction to start the passive leg lifting test, and an instruction to end the passive leg lifting test; and
the instruction to implement a sepsis screening and treatment, comprises one or more of: an instruction to start an organ failure evaluation process that is related to a sepsis, an instruction to input various physiological index values which are required for the organ failure evaluation process that is related to a sepsis, and an instruction to input a sepsis treatment measure.
